(19) Europäisches Patentamt
European Patent Office
Office européen des brevets

(11) Publication number: **0 254 086 B1**

(12) **EUROPEAN PATENT SPECIFICATION**

(45) Date of publication of patent specification: **08.04.92**

(51) Int. Cl.[5]: **C07C 49/235**, C07D 303/32, //C07D405/06,A01N43/647

(21) Application number: **87109395.1**

(22) Date of filing: **27.01.84**

(60) Publication number of the earlier application in accordance with Art.76 EPC: **0 121 979**

# (54) Heterocyclic compounds.

(30) Priority: **09.03.83 GB 8306512**

(43) Date of publication of application:
**27.01.88 Bulletin 88/04**

(45) Publication of the grant of the patent:
**08.04.92 Bulletin 92/15**

(84) Designated Contracting States:
**AT BE CH DE FR GB IT LI NL SE**

(56) References cited:

**TETRAHEDRON LETTERS, vol. 21, no. 40, 1980, pages 3849-3852, Pergamon Press Ltd, Oxford, GB; A.S. KENDE et al.: "Alkenylcuprate reagents from arenesulfonylhydrazones"**

**JOURNAL OF THE AMERICAN CHEMICAL SOCIETY, vol. 103, no. 13, 1st July 1981, pages 3837-3841, American Chemical Society, PA, Gaston, US; P.J. WAGNER et al.: "Monoradical rearrangements of the 1,4-Biradicals involved in Norrish type II photoreactions"**

(73) Proprietor: **IMPERIAL CHEMICAL INDUSTRIES PLC**
**Imperial Chemical House, Millbank**
**London SW1P 3JF(GB)**

(72) Inventor: **Clough, John Martin**
**7 Gypsy Lane**
**Marlow Bucks(GB)**
Inventor: **Lewis, Timothy**
**123 Gardner Road**
**Maidenhead Berkshire(GB)**
Inventor: **Gravestock, Michael Barry**
**72 Moss Lane Bramhall**
**Stockport Cheshire(GB)**

(74) Representative: **Alner, Henry Giveen Hamilton et al**
**Imperial Chemical Industries PLC Legal Department: Patents P.O. Box 6 Bessemer Road**
**Welwyn Garden City Herts, AL7 1HD(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**BULLETIN DE LA SOCIETE CHIMIOUE DE FRANCE, part II, no. 5, Mai 1973, pages 1676-1684, Paris, FR; G. DANA et al.: "Déshydratation des alpha-diols éthyléniques. Etude de deux séries d'aryl-1 pentène-3 diol-1,2 et d'aryl-2 hexène-4 diol-2,3"**

**CHEMICAL ABSTRACTS, vol. 92, no. 11, 17th March 1980, page 555, abstract no. 94008h, Columbus, Ohio, US; N. SHIMIZU et al.: "Silver ion-induced reaction of 1,1-dimethyl-2-trimethylsiloxyallyl chloride with 2-(p-tolyl)propene. A direct synthesis of alpha-cuparenone."**

## Description

This invention relates to ketones and oxiranes useful in the preparation of the triazole compounds having plant growth regulating and fungicidal activity described and claimed in EP-A-0 121 979

The invention provides compounds having the general formula (X):

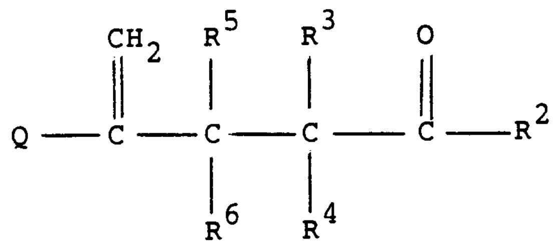

(X)

and stereoisomers thereof, wherein:

Q is phenyl substituted with one or more substituents selected from halogen, haloalkyl, $C_{2-4}$ alkyl, $C_{1-4}$ alkoxy, phenyl and phenoxy; or

Q is aralkyl having one to four carbon atoms in the alkyl moiety, optionally substituted with one or more substituents selected from halogen, haloalkyl, $C_{1-4}$ alkyl, $C_{1-4}$ alkoxy, phenyl or phenoxy; or

Q is $C_{1-4}$ alkyl; and

$R^2$, $R^3$, $R^4$, $R^5$ and $R^6$, which may be the same or different, is each H, $C_{1-6}$ alkyl, $C_{3-6}$ cycloalkyl, or is phenyl or aralkyl having one to four carbon atoms in the alkyl moiety, optionally substituted with one or more substitutents selected from halogen, haloalkyl, $C_{1-4}$ alkyl, $C_{1-4}$ alkoxy, phenyl and phenoxy; provided that Q is not 4-Chlorophenyl.

The invention further provides compounds having the general formula (XI):

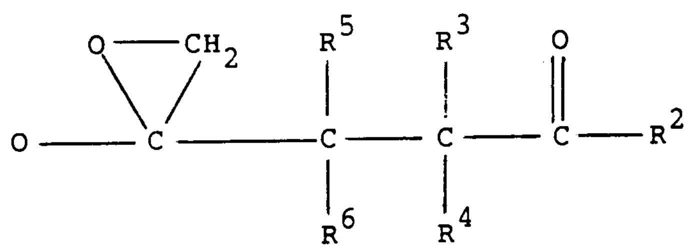

(XI)

and stereoisomers thereof, wherein:

Q, $R^2$, $R^3$, $R^4$ , $R^5$ and $R^6$ are as defined in Claim 1, provided that Q is not 4-chlorophenyl.

The compounds of the invention contain at least 2 chiral centres; consequently, each compound has at least 4 stereoisomeric forms, and these can be separated into individual isomers by methods known in the art, and this invention embraces such isomers.

Examples of suitable substitutent groups for Q, $R^2$, $R^3$, $R^4$, $R^5$ and $R^6$ when they represent aralkyl or aryl, especially benzyl or phenyl, are halogen, haloalkyl, $C_{1-4}$ alkyl, $C_{1-4}$ alkoxy (especially containing 1 to 4 carbon atoms), phenyl and phenoxy. Phenyl is preferred to benzyl.

Suitably the aryl, especially phenyl, is unsubstituted or substituted with 1, 2, or 3 ring substitutents, which may be the same or different, as defined above. Examples of Q, $R^1$, $R^2$, $R^3$, $R^4$, $R^5$ and $R^6$ are phenyl, 2-, 3- or 4-chlorophenyl, 2,4- or 2,6-dichlorophenyl, 2,4- or 2,6-difluorophenyl, 2- 3-or 4-fluorophenyl, 2-, 3- or 4-fluorophenyl, 2-, 3- or 4-bromophenyl, 2-, 3- or 4-mehoxyphenyl, 2,4-dimethoxyphenyl, 2-, 3- or 4-ethoxyphenyl, 2-fluoro-4-chlorophenyl, 2-chloro-4-fluorophenyl, 2-, 3- or 4-methylphenyl, 2-, 3- or 4-ethylphenyl, 2-, 3- or 4-trifluoromethylphenyl, 4-phenylphenyl (4-biphenylyl), 2-chloro-4-methoxyphenyl, 2-fluoro-4-methoxyphenyl, 2-chloro-4-methylphenyl, 2-fluoro-4-methylphenyl, 4-isopropylphenyl, 2-methyl-4-chlorophenyl or 2-methyl-4-fluorophenyl.

When Q, $R^2$, $R^3$, $R^4$, $R^5$ or $R^6$ is alkyl it can be a straight or branched chain alkyl group having 1 to 4 carbon atoms; examples are methyl, ethyl, propyl (n- or iso-propyl) and butyl (n-, sec-, iso- or t-butyl). When Q is alkyl, it is preferably t-butyl.

Olefins of general formula:

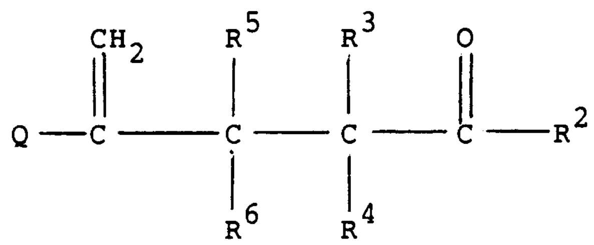

(X)

wherein Q, $R^2$, $R^3$, $R^4$ , $R^5$ and $R^6$ are as defined above, when treated with a halogen (such as bromine or iodine), a base (such as pyridine) and an alcohol of general formula $R^1OH$, wherein $R^1$ is as defined above, either in a suitable solvent, or with an excess of the alcohol forming the solvent , can be converted into halides of formula (VII):

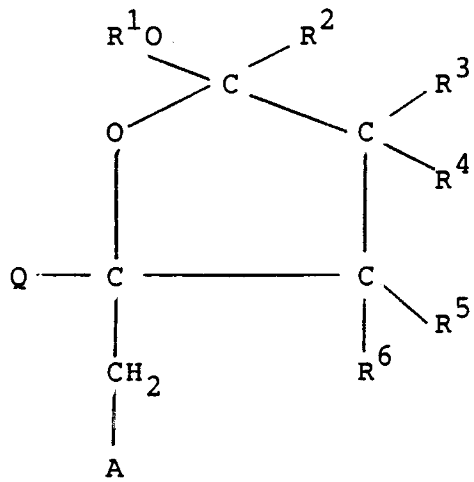

(VII)

wherein Q, $R^2$, $R^3$, $R^4$, $R^5$ and $R^6$ are as defined above, and A is a halogen atom.

Halides of general formula (VII) can then be converted into compounds of general formula (I) of EP-A-0 121 979 (see below) by treatment either with 1,2,4-triazole or with imidazole, each in the presence of an acid-binding agent or in the form of one of its alkali metal salts, in a convenient solvent (such as dimethylformamide or acetonitrile) and at a convenient temperature.

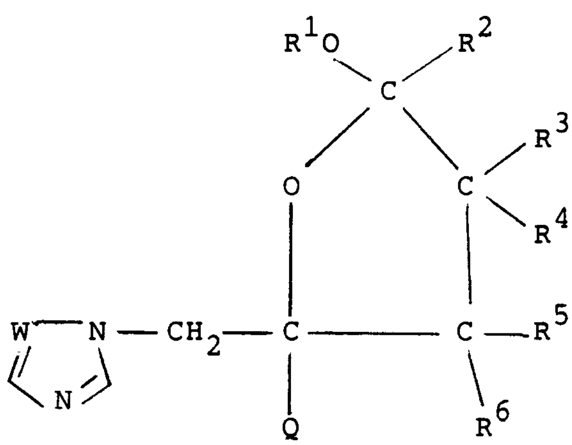

(I)

The olefins of general formula X can be prepared by methods described in the literature.

Epoxides of general formula (XI):

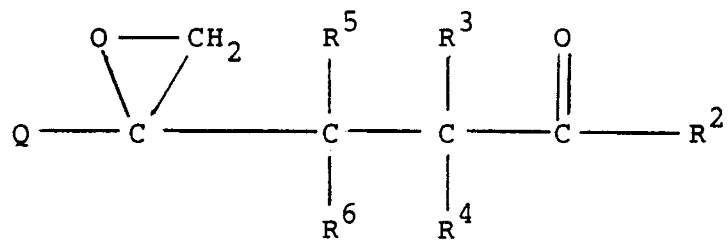

(XI)

wherein Q, $R^2$, $R^3$, $R^4$, $R^5$ and $R^6$ are as defined above can be converted to the compounds of formula I of EP-A-0 121 979 wherein $R^1$ is a hydrogen atom by treatment either with l,2,4-triazole or with imidazole, each in the presence of an acid-binding agent or in the form of one of its alkali metal salts, in a convenient solvent (such as dimethylformamide or acetonitrile) and at a convenient temperature.

Epoxides of general formula (XI) can be prepared from olefins of general formula (X) by treatment with an appropriate oxidising agent (a peracid, for example) by methods described in the chemical literature.

EXAMPLE 1

This Example illustrates the prepration of 2(2,4-dichlorophenyl)hex-1-en-5-one and also the preparation of 2(2,4-dichlorophenyl)-2-(1,2,4-triazol-l-yl)methyl-5-metho xy-5-methyltetrahydrofuran (compound No. 31 of Table I of EP-A-0 121 979).

A solution of 4-(2,4-dichlorophenyl)pent-4-enol (26.0g) in dry diethyl ether es added dropwise to a stirred solution of methylmagnesium iodide [from magnesium (4.09g) and methyl iodide (24.14g)] in diethyl ether (total volume of diethyl either ca. 200ml). Following the addition, the reaction mixture was stirred at room temperature for 1 hour then poured into a mixture of ice and dilute hydrochloric acid, and extracted with ether. The extracts were washed with water, dried over magnesium sulphate, concentrated under reduced pressure, and chromatographed on a column of silica gel (-eluting with dichloromethane: 60-80° petrol, 1:1) to give 2(2,4-dichlorophenyl)hexan-5-ol (19.2g, 69%) as an almost colourless oil.

A mixture of pyridinium dichromate (49g) and 2-(2,4-dichlorophenyl)hexan-5-ol (16g) in dry dimethylformamide (100ml) was stirred overnight at room temperature then poured into water and extracted with ether. the extracts were washed successively with dilute hydrochloric acid and water, then dried over magnesium sulphate and concentrated under reduced pressure to give almost pure 2-(2,4-dichlorophenyl)hex-1-en-5-one (14.5g, 91%) as a yellow oil, $^1H$ n.m.r. ($CDCl_3$) : $\delta$2.18 (3H,s), 2.4-3.0 (4H,m), 5.15 (1H,s), 5.41 (1H,s), 7.2-7.6 (3H,m).

By the two step method described in Example 3 of EP-A-0 121 979 for the conversion of 4-(2,4-dichlorophenyl)pent-4-enol into 2-(2,4-dichlorophenyl)-2(1,2,4-triazol-l-yl)methyl-5-methoxytetrahydrofuran, 2-(2,4-dichlorophenyl)hex-1-en-5-one was converted into the final triazole compound (52% over both steps), a white solid, m.p. 126-151°C, a 2:1 mixture of diastereomers. Selected proton n.m.r. data ($CDCl_3$): Major diastereomer: $\delta$1.57 (s,C-$CH_3$), 3.44(s,$OCH_3$). Minor diastereomer: $\delta$1.42 (s,C-$CH_3$), 3.20 (s,$OCH_3$).

EXAMPLE 2

This Example illustrates the preparation of 2-(2,4-dichlorophenyl)-2-91,2,4-triazol-l-yl)methyl-5-hydroxy-5-methyltetrahydrofuran (compound No. 30 of Table I of EP-A-0 121 979).

A solution of 2-(2,4-dichlorophenyl)hex-l-en-5-one (0.50g, prepared as described in Example 1 above) in dry dichloromethane (15ml) was cooled to 0°C and 3-chloroperbenzoic acid (0.38g) was added. The mixture was stirred at 0°C for 1 hour and then at room temperture for a further 4 hours. It was then diluted with ether, washed successively with aqueous sodium hydroxide and water, dried over magnesium sulphate, and concentrated under reduced pressure to give a pale yellow oil (0.44g) consisting mainly of 2-(2,4-dichlorophenyl)-1,2-epoxyhexan-5-one, $^1H$ n.m.r. ($CDCl_3$): $\delta$2.11 (3H,s), 2.79 and 3.06 (each 1H, d J 6 H2). A second experiment using the same procedure gave a further sample of the crude epoxide (1.58g from 1.5g of olefin) which was combined with the first batch.

The crude epoxide (1.90g) was added to a solution of sodium triazole [from 1,2,4-triazole (1.24g) and sodium hydride (0.43g)] in dry dimethylformamide (50ml). The resulting mixture was heated at 90°C for 2 hours then allowed to cool, poured into water and extracted with ethyl acetate. The extracts were washed with water, dried over magnesium sulphate, and concentrated under reduced pressure to give a yellow solid (1.8g). Trituration with diethyl ether left a white solid [1.06g, 42% from 2-(2,4-dichlorophenyl)hex-l-en-5-one], m.p. 155-160 °C, a 3:2 mixture of diasteromers of the title compound, $^1H$ n.m.r. ($CDCl_3$): δ1.44 s, $CH_3$ of major diastereomer; δ1.55 s, $CH_3$ of minor diastereomer. The infrared spectrum (nujol mull: 3200 $cm^{-1}$ but no C = O absorption) confirmed that the solid product is in the form of the cyclic hemiketal.

## Claims

**1.** Compounds having the general formula (X):

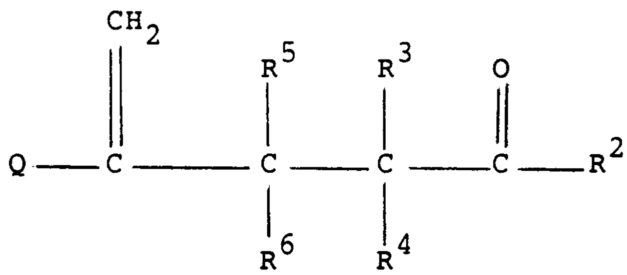

(X)

and stereoisomers thereof, wherein:
Q is phenyl substituted with one or more substituents selected from halogen, haloalkyl, $C_{2-4}$ alkyl, $C_{1-4}$ alkoxy, phenyl and phenoxy; or
Q is aralkyl having one to four carbon atoms in the alkyl moiety, optionally substituted with one or more substitutents selected from halogen, haloalkyl, $C_{1-4}$ alkyl, $C_{1-4}$ alkoxy, phenyl or phenoxy; or
Q is $C_{1-4}$ alkyl; and
$R^2$, $R^3$, $R^4$, $R^5$ and $R^6$, which may be the same or different, is each H, $C_{1-6}$ alkyl, $C_{3-6}$ cycloalkyl, or is phenyl or aralkyl having one to four carbon atoms in the alkyl moiety, optionally substituted with one or more substituents selected from halogen, haloalkyl, $C_{1-4}$ alkyl, $C_{1-4}$ alkoxy, phenyl and phenoxy, provided that Q is not 4-chlorophenyl.

**2.** Compounds having the general formula (XI):

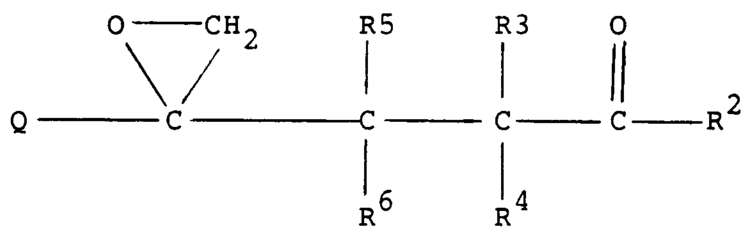

(XI)

and stereoisomers thereof, wherein:
Q, $R^2$, $R^3$, $R^4$ , $R^5$ and $R^6$ are as defined in Claim 1, provided that Q is not 4-chlorophenyl.

**3.** A compound according to Claim 1 or 2, in which Q is halosubstituted phenyl.

**4.** A compound according to Claim 3, in which Q is a dihalophenyl group.

**5.** A compound according to Claim 4, in which Q is a dichlorophenyl group.

**6.** A compound according to Claim 5, in which Q is 2,4-dichlorophenyl.

**7.** A compound according to any of Claims 1 to 6 in which $R^3$, $R^4$, $R^5$ and $R^6$ is each hydrogen.

**8.** A compound according to any of Claims 1 to 7, in which $R^2$ is hydrogen, methyl or ethyl.

## Revendications

**1.** Composés répondant à la formule générale (X) :

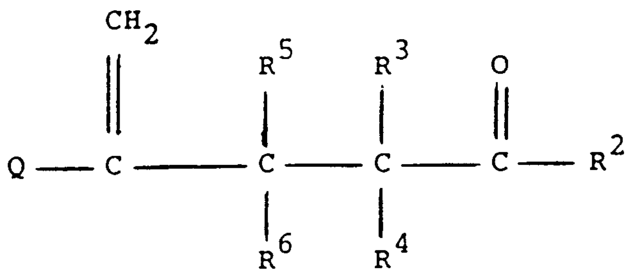

(X)

et leurs stéréo-isomères, formule dans laquelle :

Q représente un groupe phényle portant un ou plusieurs substituants choisis entre un halogène, des groupes halogénalkyle, alkyle en $C_2$ à $C_4$, alkoxy en $C_1$ à $C_4$, phényle et phénoxy ; ou

Q représente un groupe aralkyle ayant 1 à 4 atomes de carbone dans le groupement alkyle, portant facultativement un ou plusieurs substituants choisis entre un halogène, un groupe halogénalkyle, alkyle en $C_1$ à $C_4$, alkoxy en $C_1$ à $C_4$, phényle ou phénoxy ; ou

Q représente un groupe alkyle en $C_1$ à $C_4$ ; et

$R^2$, $R^3$, $R^4$, $R^5$ et $R^6{}_1$ qui peuvent être identiques ou différents, représentent chacun H, un groupe alkyle en $C_1$ à $C_6$, cycloalkyle en $C_3$ à $C_6$ ou bien un groupe phényle ou aralkyle ayant 1 à 4 atomes de carbone dans le groupement alkyle, portant facultativement un ou plusieurs substituants choisis entre un halogène, des groupes halogénalkyle, alkyle en $C_1$ à $C_4$, alkoxy en $C_1$ à $C_4$, phényle et phénoxy ; sous réserve que Q ne représente pas un groupe 4-chlorophényle.

**2.** Composés répondant à la formule générale (XI):

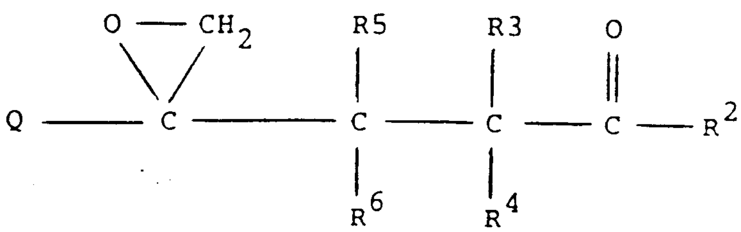

(XI)

et leurs stéréo-isomères, formule dans laquelle :

Q, $R^2$, $R^3$, $R^4$, $R^5$ et $R^6$ répondent aux définitions suivant la revendication 1, sous réserve que Q ne représente pas un groupe 4-chlorophényle.

**3.** Composé suivant la revendication 1 ou 2, dans lequel Q représente un groupe phényle substitué avec un halogène.

**4.** Composé suivant la revendication 3, dans lequel Q représente un groupe dihalogénophényle.

**5.** Composé suivant la revendication 4, dans lequel Q représente un groupe dichlorophényle.

**6.** Composé suivant la revendication 5, dans lequel Q représente un groupe 2,4-dichlorophényle.

**7.** Composé suivant l'une quelconque des revendications 1 à 6, dans lequel $R^3$, $R^4$, $R^5$ et $R^6$ représentent chacun l'hydrogène.

**8.** Composé suivant l'une quelconque des revendications 1 à 7, dans lequel $R^2$ représente l'hydrogène ou un groupe méthyle ou éthyle.

## Patentansprüche

**1.** Verbindungen der allgemeinen Formel (X)

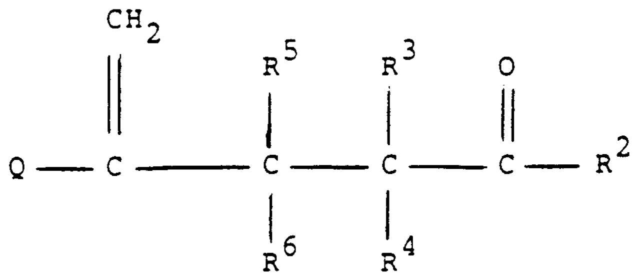

(X)

und Stereoisomere davon, worin
Q für Phenyl steht, das mit einem oder mehreren Substituenten substituiert ist, die ausgewählt sind aus Halogen, Halogenoalkyl, $C_{2-4}$-Alkyl, $C_{1-4}$-Alkoxy, Phenyl und Phenoxy; oder
Q für 1 bis 4 Kohlenstoffatome in Alkyl-Teil aufweisendes Aralkyl steht, das gegenbenenfalls mit einem oder mehreren Substituenten substituiert ist, die ausgewählt sind aus Halogen, Halogenoalkyl, $C_{1-4}$-Alkyl, $C_{1-4}$-Alkoxy, Phenyl und Phenoxy; oder
Q für $C_{1-4}$-Alkyl steht; und
$R^2$, $R^3$, $R^4$, $R^5$ und $R^6$, welche gleich oder verschieden sein können, jeweils für H, $C_{1-6}$-Alkyl oder $C_{3-6}$-Cycloalkyl oder für Phenyl oder 1 bis 4 Kohlenstoffatome im Alkyl-Teil aufweisendes Aralkyl, das gegenbenenfalls mit einem oder mehreren Substituenten substituiert ist, die ausgewählt sind aus Halogen, Hologenoalkyl, $C_{1-4}$-Alkyl, $C_{1-4}$-Alkoxy, Phenyl und Phenoxy, stehen;
mit der Maßgabe, daß Q nicht für 4-Chlorophenyl steht.

**2.** Verbindungen der allgemeinen Formel (XI)

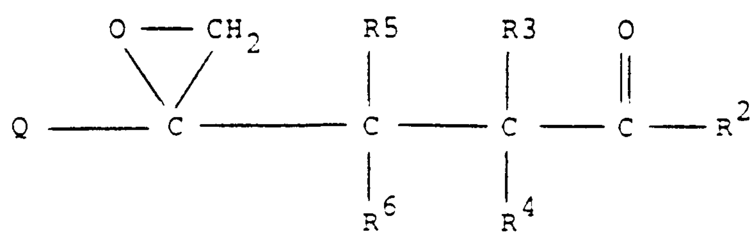

(XI)

und Stereoisomere davon, worin
Q, $R^2$, $R^3$, $R^4$, $R^5$ und $R^6$ die in Anspruch 1 angegebenen Bedeutungen besitzen, mit der Maßgabe, daß Q nicht für 4-Chlorphenyl steht.

**3.** Verbindungen nach Anspruch 1 oder 2, worin Q für halogensubstituiertes Phenyl steht.

**4.** Verbindungen nach Anspruch 3, worin Q für eine Dihalogenophenyl-Gruppe steht.

**5.** Verbindungen nach Anspruch 4, worin Q für eine Dichlorophenyl-Gruppe steht.

**6.** Verbindungen nach Anspruch 5, worin Q für 2,4-Dichlorophenyl steht.

**7.** Verbindungen nach einem der Ansprüche 1 bis 6, worin $R^3$, $R^4$, $R^5$ und $R^6$ jeweils für Wasserstoff stehen.

**8.** Verbindungen nach einem der Ansprüche 1 bis 7, worin $R^2$ für Wasserstoff, Methyl oder Ethyl steht.